(19) Europäisches Patentamt
European Patent Office
Office européen des brevets

(11) Numéro de publication: **0 292 362 B1**

(12) **FASCICULE DE BREVET EUROPEEN**

(45) Date de publication de fascicule du brevet: **26.08.92**

(51) Int. Cl.$^5$: **C07D 209/48**

(21) Numéro de dépôt: **88401140.4**

(22) Date de dépôt: **10.05.88**

# (54) Procédé de préparation d'imides halogènes.

(30) Priorité: **18.05.87 FR 8706904**

(43) Date de publication de la demande:
**23.11.88 Bulletin 88/47**

(45) Mention de la délivrance du brevet:
**26.08.92 Bulletin 92/35**

(84) Etats contractants désignés:
**AT BE CH DE ES FR GB GR IT LI LU NL SE**

(56) Documents cités:
**EP-A- 0 101 785**
**DE-A- 1 518 647**
**FR-A- 2 369 261**
**CH-A- 410 898**
**FR-A- 2 359 128**
**US-A- 3 734 925**

**JOURNAL OF THE CHEMICAL SOCIETY, 1937, partie I, pages 16-36, The Chemical Society, Londres, GB; H.D.K. DREW et al.: "Chemiluminescent organic compounds. Part I. Isomeric simple and complex hydrazides of phthalic acid and mode of formation of phthalazine and isolndole rings"**

**JOURNAL OF POLYMER SCIENCES, no. 22, partie C, 1969, pages 773-784; J. IDRIS JONES: "The reaction of hydrazine with polyimides, and utility"**

(73) Titulaire: **ELF ATOCHEM S.A.**
**4 & 8, Cours Michelet La Défense 10**
**F-92800 Puteaux(FR)**

(72) Inventeur: **Bonnet, Evelyne**
**18 Grande Avenue**
**F-60260 Lamorlaye(FR)**
Inventeur: **Gurtner, Bernard**
**5 Boulevard Agutte Sembat**
**F-38000 Grenoble(FR)**

**EP 0 292 362 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne la synthèse de polyhalogénoimides et, plus particulièrement, celle d'imides dérivés d'acides dicarboxyliques halogénés comme l'acide tétrabromophtalique.

Les polyhalogénoimides, notamment les tétrabromophtalimides et bis(tétrabromophtalimides), sont des composés bien connus qui trouvent une application comme retardateurs de flamme dans de nombreuses matières inflammables, en particulier dans les matières plastiques (voir, par exemple, l'article de S.M. SPATZ et al intitulé "Some N-substituted tetrabromophtalimide fire-retardant additives" dans Industrial and Engineering Chemistry Product Research and Development, vol. 8, n° 4 (1969) pages 397-398, ainsi que les brevets US 3 873 567 et FR 2 369 261 et les demandes JP 74-045062 et 75-064337).

Cependant, les procédés de préparation décrits dans les références précitées donnent des rendements médiocres et fournissent des produits le plus souvent colorés en jaune ou qui se colorent lors de leur mise en oeuvre et confèrent ainsi aux matières plastiques (compounds ou objets moulés) une coloration rédhibitoire et inacceptable dans bon nombre d'utilisations. D'autre part, ces produits contiennent très souvent des matières qui sont volatiles aux températures de mise en oeuvre dans certains matériaux polymériques et entraînent la corrosion des moules. En outre, l'emploi pour leur préparation de solvants organiques (notamment xylène, toluène, alcool, acide acétique), sélectionnés le plus souvent en raison de leur aptitude à former avec l'eau des mélanges azéotropiques permettant d'entraîner l'eau de condensation produite par la réaction d'imidification ou à dissoudre l'anhydride d'acide dicarboxylique halogéné, nécessite des opérations coûteuses de séparation et de récupération de ces solvants, ainsi que des moyens de séchage adaptés à l'élimination des vapeurs de solvants organiques.

Les invonvénients susmentionnés se rencontrent notamment dans le cas des polyhalogénoimides dérivés d'hydrazine et d'anhydrides d'acides carboxyliques halogénés.

La disponibilité de cette dernière matière première nécessite de plus une halogénation préalable des anhydrides d'acides dicarboxyliques. Ce point constitue un handicap supplémentaire aussi bien au niveau technique qu'économique, puisque la synthèse de ces polyhalogénoimides implique la succession des étapes suivants :

- halogénation de l'anhydride d'acides carboxyliques,
- isolement et purification de l'anhydride halogéné obtenu.

Il a maintenant été trouvé qu'il n'est pas nécessaire d'isoler et de purifier l'anhydride halogéné intermédiaire et qu'il n'est pas indispensable d'utiliser un solvant organique pour dissoudre l'anhydride et/ou éliminer l'eau de condensation et qu'en opérant sans solvant organique sous certaines conditions, on peut obtenir avec des rendements très élevés des produits incolores ou très peu colorés qui, sans purification préalable, conviennent parfaitement à l'ignifugation des matières macromoléculaires, y compris celles dont la mise en oeuvre s'effectue à haute température, particulièrement au-dessus de 250 °C.

Le procédé selon l'invention qui consiste à halogéner un anhydride d'acide dicarboxylique puis à faire réagir de l'hydrazine avec le produit brut d'halogénation est caractérisé en ce que :

a) on effectue l'halogénation de l'anhydride d'acide dicarboxylique,

b) puis on fait réagir la phase précédente obtenue en a avec de l'hydrazine.

Parmi les anhydrides utilisables, on peut citer plus particulièrement :

- les anhydrides d'acides dicarboxyliques aromatiques (benzène, naphtalène, anthracène), notamment ceux de formule générale :

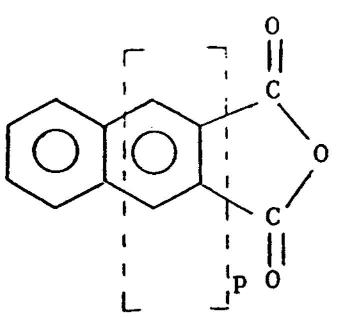

dans laquelle p est un nombre entier allant de 0 à 2.

- les anhydrides d'acides dicarboxyliques aromatiques partiellement halogénés (benzène, naphtalène, anthracène), notamment ceux de formule générale :

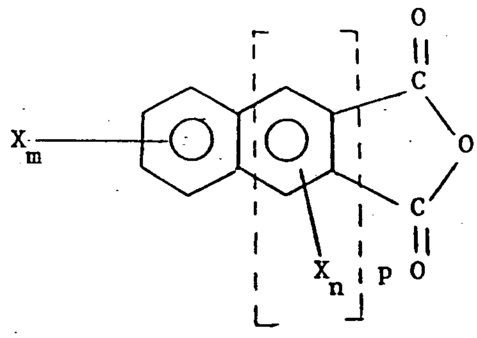

dans laquelle X est un chlore ou un brome, m varie de 0 à 4, n varie de 0 à 2, m + n est inférieur à 4 + 2p, p varie de 0 à 2. X peut aussi être un chlore et un brome (m + n supérieur à 1), c'est-à-dire qu'on peut avoir toutes les combinaisons de chlore et de brome sur la même molécule.

Conformément à la présente invention on peut utiliser un seul anhydride ou un mélange de plusieurs anhydrides. Quand on utilise un mélange d'anhydrides, un ou plusieurs d'entre eux, mais pas tous, peuvent être saturés en halogéne. On utilise de préférence l'anhydride phtalique.

L'halogénation de l'anhydride d'acide dicarboxylique, l'étape a, est réalisée suivant des techniques connues de l'homme de métier. L'halogénation consiste en une chloration ou une bromation, ou une chloration suivie d'une bromation, ou inversement. Bien qu'on puisse halogéner partiellement ou totalement, on préfère que l'halogénation soit poursuivie jusqu'à ce qu'on obtienne des produits dans lesquels m est supérieur ou égal à 2. Avantageusement on utilise un seul halogène et de préférence le brome.

On peut chlorer l'anhydride phtalique dans la chlorhydrine par exemple selon la méthode décrite dans le brevet allemand DE 1.934.174. Selon cette méthode on prépare une solution d'anhydride phtalique dans la chlorhydrine ($HSO_3Cl$), on ajoute de l'iode et on chauffe à 120 ° C, puis on traite par un courant de chlore contenant $ICl_3$. On peut aussi chlorer l'anhydride phtalique en solution dans l'oléum en présence d'un peu d'iode, par injection de chlore dans cette solution. On préfère effectuer la chloration en milieu oléum.

La bromation de l'anhydride phtalique s'effectue par exemple selon la technique décrite dans le brevet GB 1.084.375 dans lequel la bromation est réalisée en milieu oléum 65 % et catalysée par de l'iode et du fer.

La bromation de l'anhydride phtalique peut s'effectuer également selon la technique décrite dans le brevet CH 410898 dans lequel la bromation est réalisée en milieu acide sulfurique (teneur en $SO_3$ comprise entre 20 - 65 %) et en présence de chlore.

Il n'est pas nécessaire de séparer l'anhydride d'acide dicarboxylique halogéné de la phase obtenue en fin d'halogénation.

Selon l'invention on effectue l'étape b en faisant réagir la phase précédente obtenue en a avec de l'hydrazine.

L'un des avantages de l'invention apparaît clairement ici, il n'est pas nécessaire d'isoler le produit intermédiaire halogéné obtenu dans l'étape a avant de faire la réaction avec l'hydrazine. On utilise l'hydrazine sous forme d'hydrate ou de sel d'hydrazine (par exemple sulfate, hydrohalogénure, acétate). L'hydrazine ou ses dérivés peuvent s'utiliser tels quels ou sous forme de solution sulfurique. La quantité d'acide sulfurique peut varier dans de larges limites, la seule condition est qu'elle soit suffisante pour assurer la dispersion convenable des réactifs et permettre une bonne agitation. Le rapport molaire anhydride halogéné sur hydrazine est inférieur à 2, et de préférence entre 1 et 2. Cette réaction de l'étape b peut être effectuée à pression atmosphérique à des températures comprises entre 80 et 220 ° C et de préférence 110 et 200 ° C.

La durée de réaction peut varier dans de larges limites, mais est généralement comprise entre 1 et 20 heures. Après refroidissement du milieu réactionnel, la suspension solide obtenue est diluée puis filtrée et lavée à l'eau jusqu'à neutralité, puis on sèche le produit par les moyens de séchage classiques.

Selon une forme préférée de l'invention l'hydrazine sous forme d'hydrate ou de sel d'hydrazinium est mise en oeuvre sous forme de solution dans l'acide sulfurique, en introduisant progessivement cette solution dans la phase a d'anhydride halogénée préalablement obtenue ; la quantité d'acide sulfurique, ainsi que sa concentration sont calculées de telle sorte que le mélange réactionnel, à la fin de la coulée de l'hydrazine, contienne avantageusement 500 à 1500 ml de solution sulfurique par mole d'anhydride engagée en a, et de préférence 600 à 1200 ml, la concentration de cette solution étant comprise entre $H_2SO_4$ 80 % et l'oléum 65 %, et de préférence entre $H_2SO_4$ 90 % et l'oléum 40 %.

Lorsque l'étape b du procédé selon l'invention est réalisée à une température au moins égale à 130 ° C,

le produit obtenu est généralement constitué par le bis-imide répondant à la structure générale suivante :

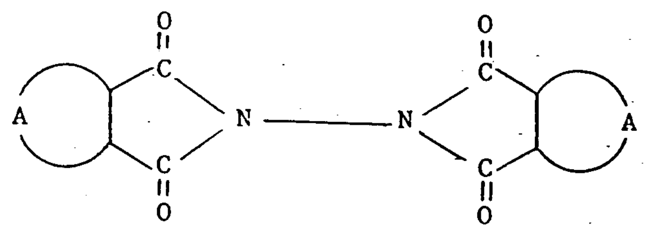

dans laquelle A représente le reste de l'anhydride halogéné mis en oeuvre.

Les produits obtenus conformément au procédé selon l'invention conviennent particulièrement bien comme retardateurs de flamme dans les matières plastiques de toute nature. Leur incorporation dans ces matières peut être effectuée par toute méthode connue à des doses allant de 5 à 40 % par rapport au poids de matière inflammable.

Les exemples suivants, dans lesquels les parties et les pourcentages sont exprimés en poids illustrent l'invention sans la limiter.

EXEMPLE 1

Dans un réacteur en verre, muni d'un agitateur et d'un dispositif de reflux, on introduit successivement :

- 148 g d'anhydride phtalique
- 1,5 g d'iode
- 700 g d'oléum 65 %

Après homogénéisation du milieu, on chauffe à 60-70 ° C et on coule en 4 heures 345 g de brome ; à la fin de la coulée, on maintient 4 heures tout en montant la température progressivement à 110 ° C.

On introduit ensuite en 2 heures une solution de 107 g de sulfate d'hydrazine dissous dans 1000 g d'$H_2SO_4$ 96 %, tout en montant la température progressivement de 110 à 180 ° C, et le milieu réactionnel est maintenu à cette température durant environ 11 heures.

Après refroidissement, dilution à l'eau, filtration et lavage du solide obtenu jusqu'à neutralité puis séchage, on obtient 430 g d'un solide blanc (rendement = 93 %) dont le spectre infra-rouge correspond à la formule :

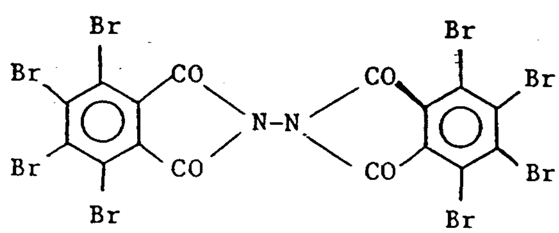

EXEMPLE 2

Dans le même appareillage qu'à l'exemple précédent, on introduit successivement :

- 148 g d'anhydride phtalique
- 1,5 g d'iode
- 700 g d'oléum 65 %.

Après dissolution de l'anhydride phtalique, on chauffe à 40 ° C, c'est-à-dire à la température d'ébullition du milieu réactionnel, et on injecte le chlore à un débit de 90 g/h ; on maintient cette introduction de chlore durant 5 heures, tout en augmentant progressivement la température jusqu'à 130 ° C.

Après refrodissement, on coule en deux heures une solution de 90 g de sulfate d'hydrazine dans 900 g d'acide sulfurique 96 %, tout en chauffant progressivement à 100-110 ° C ; après introduction de l'hydrazine, on monte la température à 170 ° C et maintient ainsi durant 11 heures.

On refroidit ensuite le mélange réactionnel, dilue à l'eau, filtre et lave le solide obtenu jusqu'à neutralité

; après séchage, on obtient, avec un rendement pondéral de 92 %, un solide blanc dont le spectre infrarouge correspond à la formule :

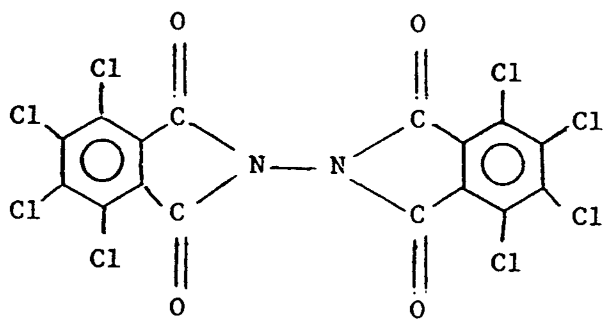

## Revendications

1. Procédé de préparation de bisimides halogénés à partir d'halogène d'anhydride d'acide dicarboxylique de formule

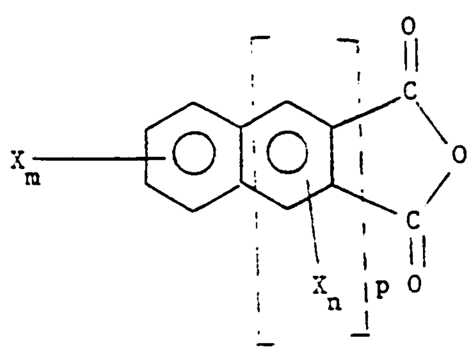

dans laquelle :
- X représente un atome de chlore ou de brome,
- n, m et p sont des nombres entiers,
- m varie de 0 à 4,
- n et p varient de 0 à 2,
- m + n est inférieur à 4 + 2p,
- X peut être un chlore et un brome si m + n > 1 ou

un mélange de tels anhydrides,

et d'hydrazine caractérisé en ce que sans utilisation d'un solvant organique :

a) on effectue l'halogénation de l'anhydride d'acide dicarboxylique,

b) puis on fait réagir la phase précédente obtenue en a avec de l'hydrazine utilisée dans un rapport molaire anhydride halogéné sur hydrazine inférieur à 2, à une température comprise entre 80 °C et 220 °C.

2. Procédé selon la revendication 1 caractérisé en ce que l'anhydride est de préférence de l'anhydride phtalique.

3. Procédé selon la revendication 1 caractérisé en ce que l'halogène est de préférence du brome.

4. Procédé selon l'une des revendications 1 à 3 caractérisé en ce que dans l'étape a on effectue une halogénation telle que m soit supérieur ou égal à 2.

5. Procédé selon l'une des revendications 1 à 4 caractérisé en ce que l'hydrazine est utilisée de préférence sous forme de sulfate d'hydrazine dissous dans l'acide sulfurique.

6. Procédé selon l'une des revendications 1 à 5 caractérisé en ce que l'étape b est effectuée entre 110 ° C et 220 ° C.

**Claims**

1. Process for the preparation of halogenated bisimides from halogen, dicarboxylic acid anhydride of formula

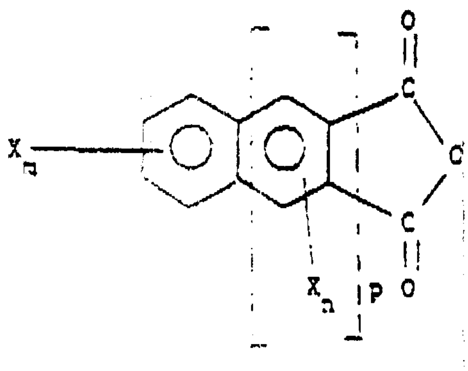

in which:
- X denotes a chlorine or bromine atom,
- n, m and p are integers,
- m varies from 0 to 4,
- n and p vary from 0 to 2,
- m + n is smaller than 4 + 2p, and
- X may be a chlorine and a bromine if m + n > 1

or a mixture of such anhydrides and hydrazine, characterised in that without using an organic solvent:

a) halogenation of the dicarboxylic acid anhydride is carried out,

b) the preceding phase obtained in a is then reacted with hydrazine used in a molar ratio of halogenated anhydride to hydrazine of less than 2, at a temperature between 80 ° C and 220 ° C.

2. Process according to Claim 1, characterised in that the anhydride is preferably phthalic anhydride.

3. Process according to Claim 1, characterised in that the halogen is preferably bromine.

4. Process according to one of Claims 1 to 3, characterised in that in stage a a halogenation is carried out such that m is greater than or equal to 2.

5. Process according to one of Claims 1 to 4, characterised in that hydrazine is preferably employed in the form of hydrazine sulphate dissolved in sulphuric acid.

6. Process according to one of Claims 1 to 5, characterised in that stage b is carried out between 110 ° C and 220 ° C.

**Patentansprüche**

1. Verfahren zur Herstellung von halogenierten Bisimiden ausgehend von Halogendicarbonsäureanhydriden der Formel

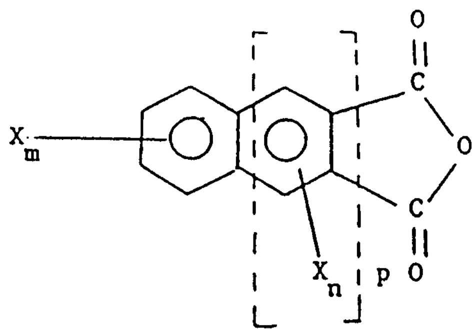

in der

- X ein Chloratom oder Bromatom,
- n, m und p ganze Zahlen,
- m von 0 bis 4,
- n und p von 0 bis 2 bedeuten, wobei
- m + n niedriger als 4 + 2p ist und
- X Chlor oder Brom sein kann, wenn m + n > 1 ist,

oder von einer Mischung von solchen Anhydriden, und von Hydrazin, dadurch gekennzeichnet, daß man ohne Verwendung eines organischen Lösungsmittels

a) die Halogenierung des Dicarbonsäureanhydrids ausführt,

b) dann die nach a) erhaltene Phase mit Hydrazin bei einem Molverhältnis des halogenierten Anhydrids zu dem Hydrazin kleiner als 2 und bei einer Temperatur im Bereich zwischen 80° und 220° C umsetzt.

**2.** Verfahren nach Anspruch 1, dadurch gekennzeichnet, daß das Anhydrid vorzugsweise Phthalsäureanhydrid ist.

**3.** Verfahren nach Anspruch 1, dadurch gekennzeichnet, daß das Halogen vorzugsweise Brom ist.

**4.** Verfahren nach einem der Ansprüche 1 bis 3, dadurch gekennzeichnet, daß man in der ersten Stufe a) eine Halogenierung durchführt, bei der m größer oder gleich 2 ist.

**5.** Verfahren nach einem der Ansprüche 1 bis 4, dadurch gekennzeichnet, daß das Hydrazin vorzugsweise in Form von in Schwefelsäure gelöstem Hydrazinsulfat eingesetzt wird.

**6.** Verfahren nach einem der Ansprüche 1 bis 5, dadurch gekennzeichnet, daß die Stufe b) zwischen 110° C und 220° C durchgeführt wird.